# EUROPEAN PATENT APPLICATION

(11) **EP 3 431 121 A1**
(43) Date of publication of application: **23.01.2019**
(21) Application number: 17182266.1
(22) Date of filing: 20.07.2017
(51) Int. Cl.: A61M 5/142, A61M 5/145, A61M 5/148, A61M 5/168

(54) **A MEDICAL FLUID TRANSFER SYSTEM AND METHOD OF USE**

(71) Applicant: Epsilon Elektronik Sanayi ve Ticaret A.S., 34360 Istanbul (TR)
(72) Inventor: TUNCEL, Mehmet Tunc, 34360 Istanbul (TR); BAYHAN, Hikmet, 34360 Istanbul (TR)
(74) Representative: Sevinç, Erkan

(57) **Abstract**

The present invention proposes a medical fluid transfer system (100) for use in a fluid transfer process that transfers selected amounts of fluid from a source fluid container (240) to an IV bag (170) comprising: an user interface (140) configured to receive instructions from an user; a transfer station (214) having a connector (253) configured to couple to a fluid switch (252) for controlling fluid flow; a motor (261) for arranging the position of the connector (253); a control system configure to draw at least a portion of the fluid from the source fluid container (240) by retracting a plunger on the syringe (241), and advance the plunger of the syringe (241) to drive at least a portion of the fluid towards the IV bag (170). said medical fluid transfer system (100) further comprises: a mounting module (210) for holding and actuating the syringe (241), wherein the mounting module (210), an upper flange housing (215) and a lower flange housing (213) configured to receive a portion of the plunger of the syringe (241); and a motor (290) which is in communication with the upper flange housing (215) and the lower flange housing (213) for actuating the plunger of the syringe (241). Said upper flange housing (215) and the selector lever (212) are movable such that the syringe (241) at a different size can be used in the medical fluid transfer system (100).

## Description

### Technical Field of the Invention

The present invention relates to a medical fluid system and a method for use in a fluid transfer process that transfers selected amounts of fluid from a source fluid container to a target bag.

### Background of the Invention

Cancer is the disease caused by an uncontrolled division of abnormal cells in a part of the body. Some cancers may eventually spread into other tissues. In all types of cancer, some of the body's cells begin to divide without stopping and spread into surrounding tissues. Antineoplastic or anticancer drugs are the drugs that prevent or inhibit the maturation and proliferation of neoplasms. Antineoplastic agents travel the body and destroy cancer cells.

However, the selectivity of antineoplastic drugs against cancer cells is low. Because there is not much difference between the malignant cell and the normal human cell in terms of qualitative aspect, the difference is more quantitative. Antineoplastic drugs can destroy cancer cells that are multiplying in a pathological way in the body, as well as normal cells that multiply rapidly (such as embryo and fetal cells, intestinal and oral mucosal proteins, bone marrow cells).

To reduce this carcinogenic effect to a minimum degree, the amount of drug should be adjusted precisely according to the weight and size of the patient. To maintain the patient's health, the fluid balance in the intracellular and extracellular spaces needs to remain relatively constant. In the prior art, a large number of bags need to be filled with customized amounts and types of drags on a daily basis for many different patients. If performed manually, this task is susceptible to mistakes by healthcare technicians. It can also continuously expose healthcare technicians to harmful medicinal vapors, and it can be a physically difficult chore for a healthcare technician, especially when using a large-diameter filling syringe in which the syringe plunger is especially wide and hard to push forward and back.

In the prior art, it is know that a medical fluid transfer devices is used for fluid transfer from a source container to a target bag when there is a need of potentially dangerous fluids transfer. Such devices replenish the serum bag with respect to the prescription indicated by the doctor before use. Said devices also allow the drug to be reconstituted by transferring the drug to the vial according to the type of the drug.

It is vital to prepare the medication by transferring the prescription drug to the IV bag in the correct amount. By associating the prepared medicine bag with the patient concerned, it is ensured that it is transferred to the right patient. In addition, if the healthcare personnel transferring the medicinal product are exposed to it during the procedure, the medicinal product is exposed to the carcinogenic effect of the medicament. Every day, the health personnel who perform this procedure are likely to make mistakes. For this reason, during the preparation of medicines, automated systems are used to prepare the medicines for the defined patient in a closed system without removing the medicines

A prior art reference, among many others, relevant to the technical field of the present invention is the US2008172021 (A1**)** publication. It discloses a device for dispensing fluid in a dosed manner, comprising a housing, a pressure bag and connecting means, the housing comprising a carrying device for suspending the pressure bag and pumping means for pumping pressure fluid into the pressure bag, a measuring device being provided for measuring, during use, the weight or changes therein of the pressure bag with a fluid container included therein. In such automated systems, mainly two syringe processors is used, one of them is used for high volume liquid transfer designed such as a 60 ml syringe and the other one is used for low volume liquid transfer such as a 20 ml syringe. Both syringes are not used at the same time (only one system is required) therefore, two syringe processors that cannot be used on the same system increase the cost. There is a need of a device for dispensing fluid which allow to usage of the low and high volume syringes (20 and 60 ml) with only a single processor.

In the prior art, a fluid transfer station and a weight sensor is arranged separated and have a communication link established between them, such as by cable. Further, the fluid transfer station can be configured to transfer fluid from a vial to an IV bag using a peristaltic pump which is also separated as a different unit which increases complexity. Many such automated devices for dispensing fluid use a wired hand-reader in clean room condition. The use of a wired hand-reader in a clean room condition is difficult.

The transfer of liquid in the desired volume can be prevented since the vial injector in the aforementioned systems automatically directs the IV bag from the syringe but allows the valves to be closed before the drop of pressure in the system at the end of the syringe withdrawal or dispensing operation. There is a need of a system which allows the liquid transfer to be done precisely not affecting by the pressure changes in the system. Current fluid transfer devices and methods in the medical field suffer from various drawbacks, including high cost and not enabling selection of different type of syringes.

### Summary of the Invention

The present invention proposes a medical fluid transfer system for use in a fluid transfer process that transfers selected amounts of fluid from a source fluid container to an IV bag comprising: an user interface configured to receive instructions from an user; a transfer station having a connector configured to couple to a fluid switch for controlling fluid flow; a motor for arranging the position of the connector; a control system configure to draw at least a portion of the fluid from the source fluid container by retracting a plunger on the syringe, and advance the plunger of the syringe to drive at least a portion of the fluid towards the IV bag. Said medical fluid transfer system further comprises: a mounting module for holding and actuating the syringe, wherein the mounting module includes a selector lever with at least two mounting portions which can be selected with respect to the size of the syringe, an upper flange housing configured to receive a portion of a plunger of the syringe, and a lower flange housing configured to receive a portion of the plunger of the syringe; and a motor which is in communication with the upper flange housing and the lower flange housing for actuating the plunger of the syringe in that said upper flange housing and the selector lever are movable such that the syringe at a different size can be used in the medical fluid transfer system.

In an alternative embodiment, the mounting module comprises at least one fluid detector configured to detect a presence or absence of fluid in the fluid switch in that said at least one fluid detector is provided with a fluid detector housing.

In an alternative embodiment, the mounting module comprises at least one presence detector configured to detect a presence or absence of the syringe in that said at least one presence detector is provided with a presence detector housing.

In an alternative embodiment, the connector comprises a female shaft which can be brought into two different positions wherein a first position of the connector is configured to allow fluid to flow from the source fluid container to the syringe and block fluid from flowing from the syringe to the source fluid container, a second position of the connector is configured to allow fluid to flow from the syringe to the IV bag and block fluid from flowing from the IV bag to the syringe.

In an alternative embodiment, the selector lever comprises a first mounting portion and a second mounting portion wherein the first mounting portion and the second mounting portion have a U shaped portion such that, in use, a body flange of the syringe can sit on said U shaped portion.

In an alternative embodiment, each of the upper flange housing and the lower flange housing has a fixing member configure to apply pressure to a plunger flange of the syringe and prevent the syringe from disengaging from the mounting module.

In an alternative embodiment, the medical fluid transfer system further comprises a shaker configured to mix, blend, or to agitate substances in the source fluid container by shaking.

In an alternative embodiment, the shaker has a mounting bracket that allows the source fluid container to be held.

In an alternative embodiment, the medical fluid transfer system further comprises a positive displacement pump for transferring of fluid between the first fluid container and the second fluid container or the source fluid container, having a casing, a mounting interface, a plurality of lobes, a rotor, and a pump motor wherein said positive displacement pump is configured to use with a hose assembly such that when the rotor of the positive displacement pump turns, a part of a tube of the hose assembly under compression is compressed, or occludes, thus forcing the fluid to be pumped to move through the tube. The positive displacement pump can be a peristaltic pump.

In an alternative embodiment, the selector lever lock comprises a selector lever lock configured to sense a presence or absence of the syringe in the selector lever lock wherein if the selector lever lock does not sense the presence of the syringe the medical fluid transfer system warns the user through the user interface.

In an alternative embodiment, said fluid switch has a first engagement interface configured to couple to the source fluid container, a second engagement interface coupled to an IV bag hose and a third engagement interface coupled to a syringe. In an alternative embodiment, the selector lever comprises at least two magnet sensor for sensing what type of the syringe is attached to the selector lever.

According to the present invention, a method of transferring fluid using a medical fluid transfer system comprising: receiving instructions through an user interface of the medical fluid transfer system, the instructions identifying a specified volume of fluid to transfer from a source fluid container to a IV bag; moving an upper flange housing and/or a selector lever of a mounting module with respect to the size of the syringe ; providing a fluid switch to couple to a connector of the medical fluid transfer system for controlling fluid flow, said connector comprising a female shaft movable between a first position and a second position; attaching the source fluid container, the syringe and the IV bag to the fluid switch; transferring the fluid from the source fluid container to the IV bag by a control system of the medical fluid transfer system, stopping the transfer of the fluid when the specified volume of fluid is transferred to the IV bag based on the information received from the user interface or stopping the transfer of the fluid based on the information received from a fluid detector configured to detect a presence or absence of the fluid in the fluid switch.

In an alternative embodiment, the method further comprises attaching a plunger of the syringe to a lower flange housing or a upper flange housing on the fluid transfer system with respect to the type of selected syringe, the lower flange housing and the upper flange housing being coupled to a motor and configured to move the plunger of the syringe to transfer fluid from a the source fluid container through the fluid switch and into the syringe as the plunger is withdrawn and to transfer fluid from the syringe through the fluid switch and into the IV bag as the plunger is advanced.

### Brief Description of the Figures

Accompanying drawings are given solely for the purpose of exemplifying a medical fluid transfer system and method thereof whose advantages over prior art were outlined above and will be explained in detail hereinafter:
Fig. 1 demonstrates a perspective view of a fluid transfer system having a transfer station configured to transfer fluids where a syringe, IV bag and a source fluid container are in an engaged configuration are shown according to the present invention.
Fig. 2 demonstrates a perspective view of a fluid transfer system having a transfer station configured to transfer fluids where the syringe and the IV bag are removed as shown in FIG. 1.
FIG. 3 is a front view of the system of FIG. 2.
FIG. 4 is a partial perspective view of the system of FIG. 2 showing a transfer station and a mounting module.
FIG. 5 is a front view of the system of FIG. 1 where a syringe, a source fluid container and IV bag are in an engaged configuration with a fluid switch, and a shaker is in fluid communication with a peristaltic pump.
FIG. 6 is a rear view of the system of FIG. 1.
FIG. 7 is partial perspective view of a mounting module of the system according to the present invention.
FIG. 8 shows partially perspective of a transfer station and a mounting module of the system in that a source fluid container, a syringe and an IV bag are in an engaged configuration with a fluid switch.
FIG. 9 shows the mounting module where the syringe is unattached as shown in FIG: 8.
FIG. 10a is another partial perspective view of the system according to the present invention wherein the smaller size (i.e., 20 ml) can be used.
FIG. 10b is another partial perspective of the system of the FIG: 2, wherein an upper flange housing is rotated wherein an upper flange housing is rotated to allow usage of the 50/60 ml syringe.
FIG. 10c is another partial perspective of the system of the FIG: 2, wherein the selector lever lock is rotated to allow usage of the 50/60 ml syringe.
FIG. 11 is another partial perspective view of the system according to the present invention, where a plunger actuator is extended.
FIG. 12 is a perspective view of the medical fluid transfer system shown in FIG. 2, where a first fluid container and a second container are in a communication through a positive displacement pump.
FIG: 13 is a rear perspective view of an automated actuator according the present invention.
FIG: 14 is a partially exploded view of the transfer station of the FIG: 13.
FIG: 15 is a front view of the mounting module and the connector of the FIG: 13.
FIG. 16 is another front view of the mounting module and the connector of the FIG: 13., where the female shaft is rotated to allow fluid transfer from the connector to the IV bag.

### Detailed Description of the Invention

- 100.: Fluid transfer system
- 110.: Barcode reader
- 120.: Shaker
- 121.: Mounting bracket
- 130.: Measuring unit
- 131.: Tray shaft
- 140.: User interface
- 150.: Positive displacement pump
- 151.: Mounting interface
- 160.: Printer
- 170.: IV bag
- 171.: IV bag hose

- 210.: Mounting module
- 212.: Selector lever
- 213.: Lower flange housing
- 214.: Transfer station
- 215.: Upper flange housing
- 216.: Fixing member
- 217.: First mounting portion
- 219.: Second mounting portion
- 222.: Intermediate portion
- 240.: Source fluid container
- 241.: Syringe
- 242.: Selector lever lock
- 243.: Source connector portion
- 250.: Fluid detector
- 251.: Fluid detector housing
- 252.: Fluid switch
- 253.: Connector
- 260.: Female shaft
- 261.: Motor
- 265.: Cover
- 266.: Presence detector
- 270.: Presence detector housing
- 280.: Plunger actuator
- 290.: Motor
- 291.: Motor controller

- 300.: First fluid container
- 310.: Second fluid container
- 311.: Hose assembly

- 510.: Threaded shaft

Referring now to the figures outlined above, the present invention proposes a fluid transfer device and system for transferring precise amounts of fluid from one or more source containers into one or more target container.

In the medical field, it is vital to dispense fluids in precise amounts and to transport potentially dangerous fluids. A medical fluid can be stored in a vial or other container, and a precise dosage amount of the medication can be extracted and transferred to a target bag so that the dosage amount can be delivered to a patient. To achieve the desired proportions of medical fluids, it is desired to precisely measure the amounts of fluids transferred into the target container. Also, precisely measuring the amount of fluid transferred from the source container to the target container can reduce the amount of fluid wasted. Current fluid transfer devices in the medical field suffer using different type of syringes in a same device.

According to the present invention, a medical fluid transfer system (100) for use in a fluid transfer process that transfers selected amounts of fluid from a source fluid container (240) to an IV bag (170) comprising: an user interface (140) configured to receive instructions from an user; a transfer station (214) having a connector (253) configured to couple to a fluid switch (252) for controlling fluid flow; a motor (261) for arranging the position of the connector (253); a control system configure to draw at least a portion of the fluid from the source fluid container (240) by retracting a plunger on the syringe (241), and advance the plunger of the syringe (241) to drive at least a portion of the fluid towards the IV bag (170). Said medical fluid transfer system (100) further comprises: a mounting module (210) for holding and actuating the syringe (241), wherein the mounting module (210) includes a selector lever (212) with at least two mounting portions which can be selected with respect to the size of the syringe (241), an upper flange housing (215) configured to receive a portion of a plunger of the syringe (241), and a lower flange housing (213) configured to receive a portion of the plunger of the syringe (241); and a motor (290) which is in communication with the upper flange housing (215) and the lower flange housing (213) for actuating the plunger of the syringe (241). Further, said upper flange housing (215) and the selector lever (212) are movable such that the syringe (241) at a different size can be used in the medical fluid transfer system (100). According to the present invention it is provided use of different type of syringes (241) with only a single processor of the system (100).

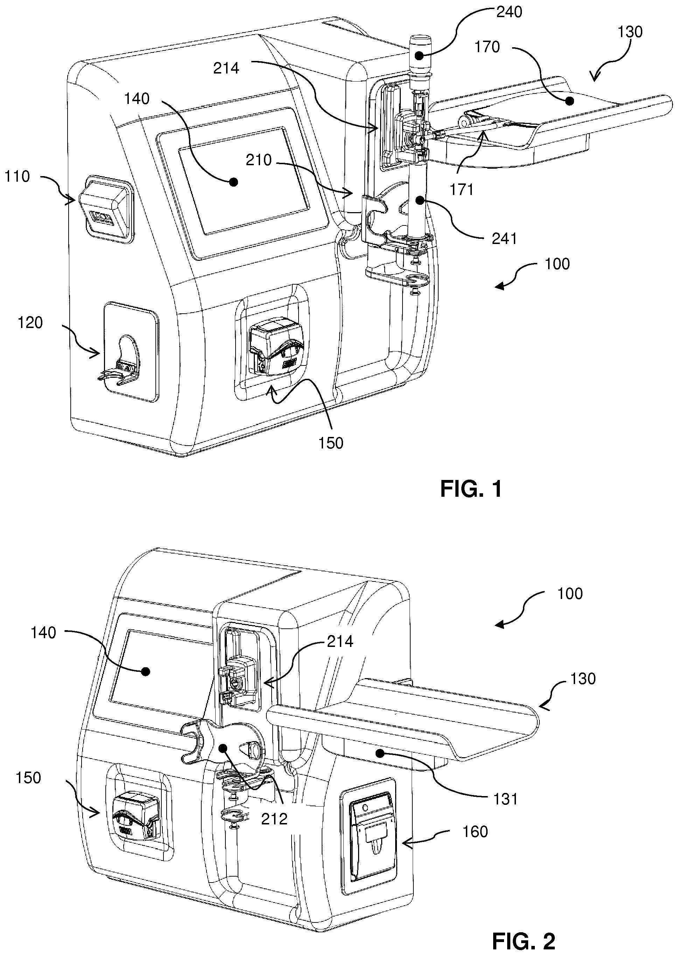

According to the present invention, FIG. 1 shows a preferred embodiment of a medical fluid transfer system (100). The fluid transfer system (100) includes a user interface (140) having a touch screen; said user interface (140) is possibly integrated into a main body of the fluid transfer device. The user interface (140) can include, for example, a display, a keypad, and/or a touch screen display. The user interface (140) can be configured to receive instructions from the user, for example, regarding the amounts of fluid to be transferred and the types of fluids to be transferred. The user interface (140) can also be configured to provide information to the user, such as error messages, alerts, or instructions (e.g., to replace an empty container). In some embodiments, the user interface (140) can also send information (e.g., results or alerts) to a remote source. Further, the user interface (140) can include one or more connection types and can be configured to allow connectivity to multiple remote sources at once. In some embodiments, the system (100) does not include a communication user interface (140) and does not communicate with the remote source.

In some embodiments, as shown in FIG: 2, the fluid transfer system (100) can have a printer (160) which is configured to print labels for use with the fluid transfer system (100). Said printer (160) can be a thermal printer which uses heat to transfer impressions to paper. When a fluid transfer is performed, the printer (160) can print a label automatically to be placed on an IV bag (170). When the transfer process is performed, the printer (160) can print a label automatically to be placed on the target container (e.g., IV bag). The label can include information such as the fluid type, the concentration the amount of fluid, the intended patient, the requesting doctor, etc. After the label is placed on the target container, said label can be read by a barcode reader (110) such that the latest information about the target container is accessible. In some embodiments, the printer (160) can be attached to the fluid transfer system (100) by a wire or cable extending from a port on the system or by a wireless data connection.

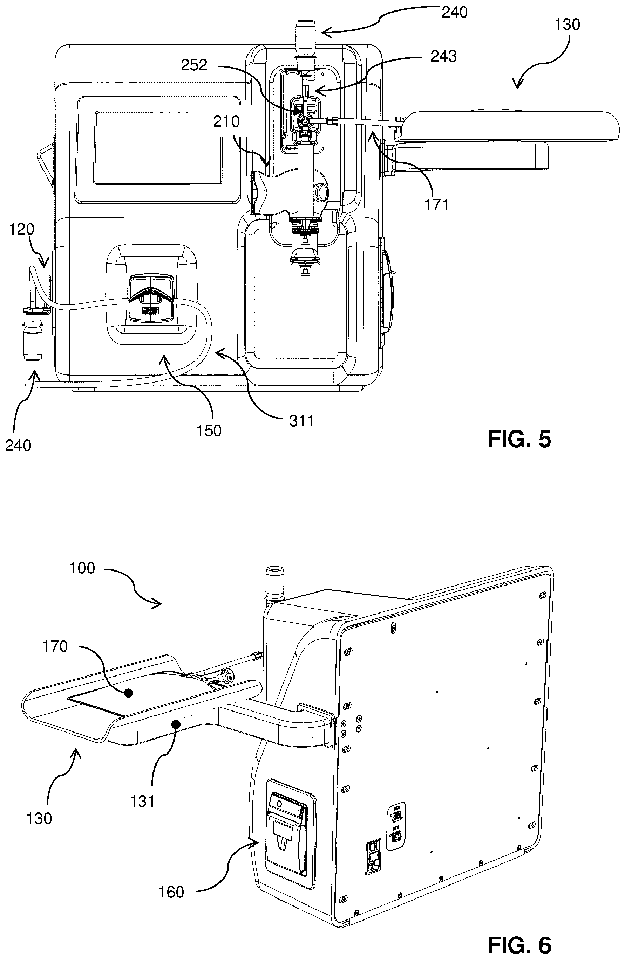

As shown in FIG. 1, the fluid transfer system (100) can include a barcode reader (110) which is an electronic device that can read and output printed barcodes to a device. When a bar codes is scanned/read by the barcode reader (110) that is in communication with a controller/processor, said barcode reader (110) identifies the types of fluid contained therein. Again in some embodiments, said fluid transfer system (100) can include a shaker (120), as shown in FIG. 1, preferably located at one lateral side of the main body. Said shaker (120) can be used to mix, blend, or to agitate substances in a source fluid container (240) (e.g., a vial) by shaking them. As shown in FIG. 5, said second fluid container (310) can be a vial (300). Said shaker (120) has a mounting bracket (121) that allows the vial or the like to be firmly held in place. In use, the source fluid container (240) (a vial) is secured to the mounting bracket and the shaker device (120) is operated to vigorously shake the source fluid container (240) along the predetermined path of travel.

Before using powdered medicaments (lyophilized) in the source fluid container or the like, it can be needed to reconstitute the necessary amount with reconstitution liquid. The reconstitution fluid can be transferred into the source fluid container (240) in two ways: manually by using a syringe (241) or by using a positive displacement pump (150) of the fluid transfer system (100). At the beginning of the both operations, the source fluid container is scanned/read with the barcode reader (110) and a medicament definition is processed by the fluid transfer system (100). According to the read data, said source fluid container (240) can be shaken with the shaker (120). When the user want to manual dilution, the manual dilution process is selected on the screen of the user interface (140). The fluid transfer system (100) retrieves information about the dilution fluid that is compatible with the medicament that the treated medicament is known from the data bank and gives the user information about which dilution fluid is competent.

The positive displacement pump (150) transfers the fluid through the hose assembly (311). The transfer rate of the positive displacement pump (150) can be changed (Rpm) and the amount of fluid transferred by the positive displacement pump (150) varies with a hose diameter of the hose of the hose assembly (311). The operation of the positive displacement pump (150) is controlled by the controller based on commands or information received from the user. The hose inner diameter can be changed from the positive displacement pump tab on the settings screen. Said positive displacement pump (150) can be used in fluid transfer into the source fluid container (240) during the lyophilized medicament reconstitution process. Further, the fluid transfer from the bulk serum to the serum bags at the desired amount (pre-filling), elastomeric pump filling and application set (fluid filling) priming process.

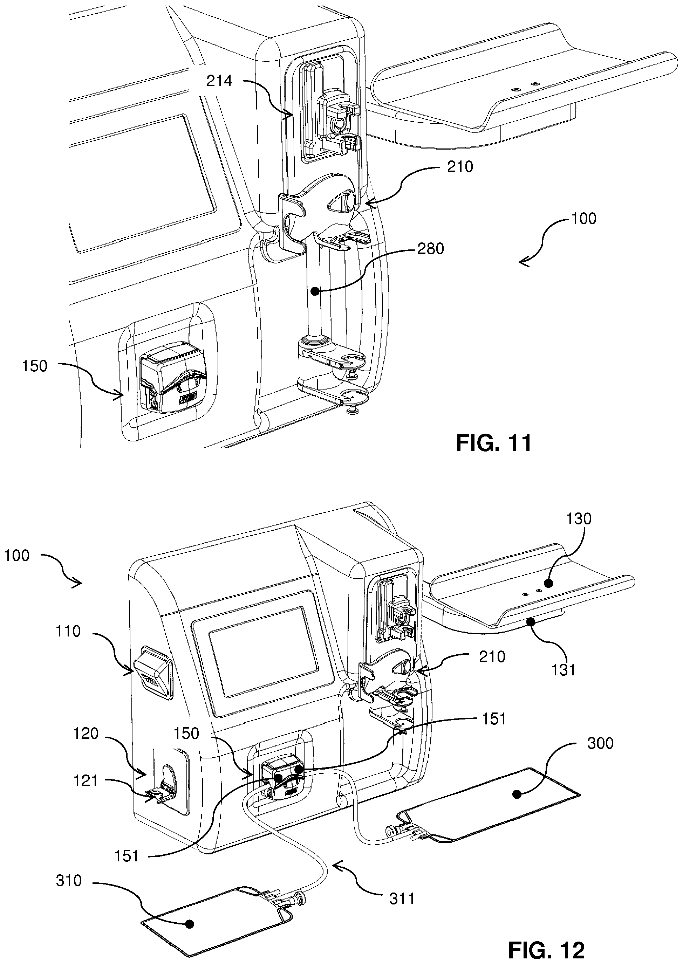

When the user wants to start the reconstitution process, "a positive displacement pump (150) option" is selected on the screen. In a preferred embodiment, the peristaltic pump is used, said peristaltic pump is a type of positive displacement pump used for pumping a variety of fluids. The control system of the fluid transfer system (100) can be configured to operate the positive displacement pump at variable speeds. Further, the positive displacement pump (150) can include a casing, a mounting interface (151), a plurality of lobes, a rotor, and a pump motor. In some embodiments said lobes of the positive displacement pump (150) can be rollers, shoes, wipers, or other members that facilitate the operation of the pump. As the rotor turns, the part of tube under compression is compressed, or occludes, thus forcing the fluid to be pumped to move through the tube. The fluid is contained within a flexible tube fitted inside a circular pump casing. Referring to FIG. 12 and 5, a hose assembly (311) is configured to be inserted within the mounting interface (151) of the positive displacement pump (150) in order to facilitate the transfer of fluid between the first fluid container (300) (source container) and the second fluid container (310) (target container). As shown in FIG. 12, a hose assembly (311) has a first connector and a second connector for attaching to the second fluid container (310) and the first fluid container (300). The first connector can be configured to removably couple to the first container and the second connector can be configured to removably couple to the second container. The fluid transferred weight is measured by taking it on the measuring unit (130) in the system. Final weight information is transferred to the data base. When the weighing process is over, the user is warned on the screen to go to the next shaker operation. At the next step, the user places the source fluid container (240) to the shaker (120).

The fluid transfer system (100) acquires knowledge of the drug-compatible diluent/ reconstitution fluid and transfer volume from the data bank that the medical fluid recognizes. The user can select the volume to be transferred on the screen and the name of the type of the fluid. After transferring the fluid into the source fluid container (240), it is necessary to shake for a certain period of time and at a certain rate for the purpose of dissolving. The system (100) retrieves data about the time and frequency of rinsing related to the medicament it is familiar with from the database. The user is informed of how long in time and speed to shake. The user starts the operation by confirming it on the screen. At the end of the process, the user checks the contents of the source fluid container (240) if necessary, the shaking operation can be repeated. For repeat shake operation, the predefined times (5 sec, 10 sec, 15 sec ...) can be selected by the user and the operation can be started after confirmation. After the shaking operation is done, it is confirmed that the operation is completed successfully on the screen. This confirmation information is sent to the data bank of the system.

In a possible embodiment, the hose assembly (311) for the transfer of medical fluids includes a hose having a proximal end and a distal end. An elastomeric portion can be disposed between the proximal end and the distal end, the elastomeric portion can have a first portion and a second portion. The second portion can be more flexible than the first portion.

A source connector portion (243) is attached to the source fluid container (240) (e.g., a vial) and the user transfers the medicament into the source fluid container (240) with the help of the syringe (241). Said source connector portion (243) can be used to transfer fluid from a source container (e.g., a vial) to the syringe (241) or any other intermediate measuring container and then to the target container. When the weighing process is finished, the user is warned on the screen to go to the shaking process. The user places the source fluid container (240) to the shaker (120). After transferring the fluid into the source fluid container (240), it is necessary to shake it for a certain period of time and at a certain rate for the complete dissolution of the drug. The fluid transfer system (100) retrieves information about the time and frequency of rinsing related to the drug it is familiar with from the database. The user will be informed of how long in time and speed to shake. The user starts the operation by confirming it on the touch screen. At the end of the process, the user checks the contents of the source fluid container (240). If necessary, the process can be repeated. For repeat shake operation, the predefined times (5 sec, 10 sec, 15 sec ...) are selected by the user and the operation is started after confirmation, keeping the speed of the shaker (120) at the same level.

Transferred weight of the medical fluid can be measured by putting the target container on a measuring unit (130). As shown in FIG. 1, the measuring unit (130) includes a tray which can be configured to support and weight the IV bag (170) or any other target container. Final weight of the IV bag (170) information is transmitted to data base of the fluid transfer system (100). In a preferred embodiment, the measuring unit (130) is attached to a tray shaft (131), which can be attached to the main body of the system. Said measuring unit (130) can include a load cell which is used as a transducer for creating an electrical signal. The measuring unit (130) is used to measure the weight of the target containers before or after the medical fluid has been transferred. The final weight of a target container can be compared to an expected weight by the controller to confirm that the proper amount of fluid was transferred into the target container. When the fluid transfer system (100) is turned on, it can be checked whether the measuring unit (130) is loaded or not. The load cell of the measuring unit (130) can have a transducer that is used to create an electrical signal whose magnitude is directly proportional to the force being measured. Whether or not there is a load on the measuring unit (130) is checked by comparing the value read from the load cell with the predefined value in the memory of the system.

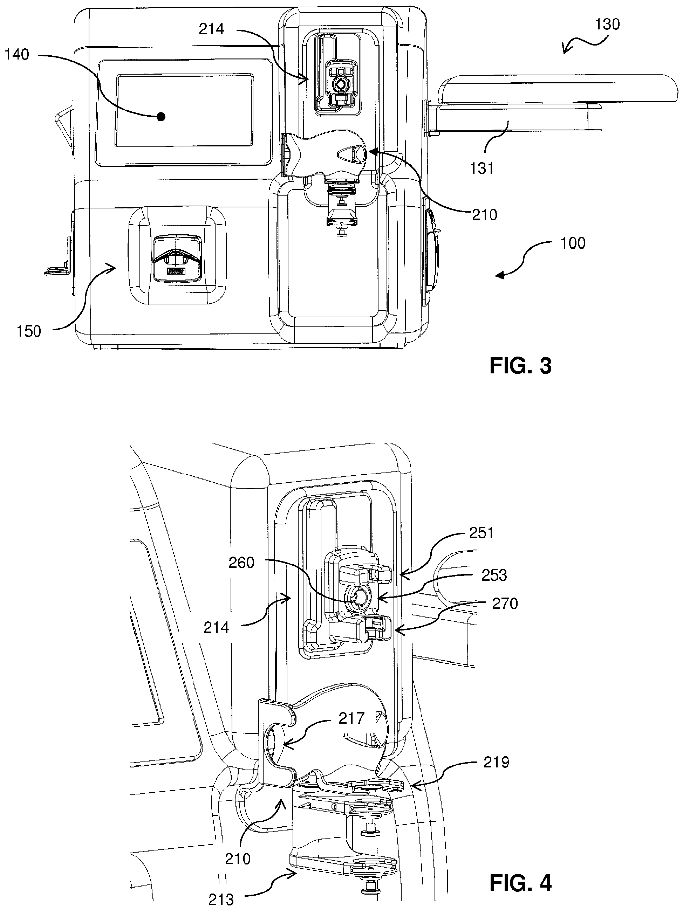

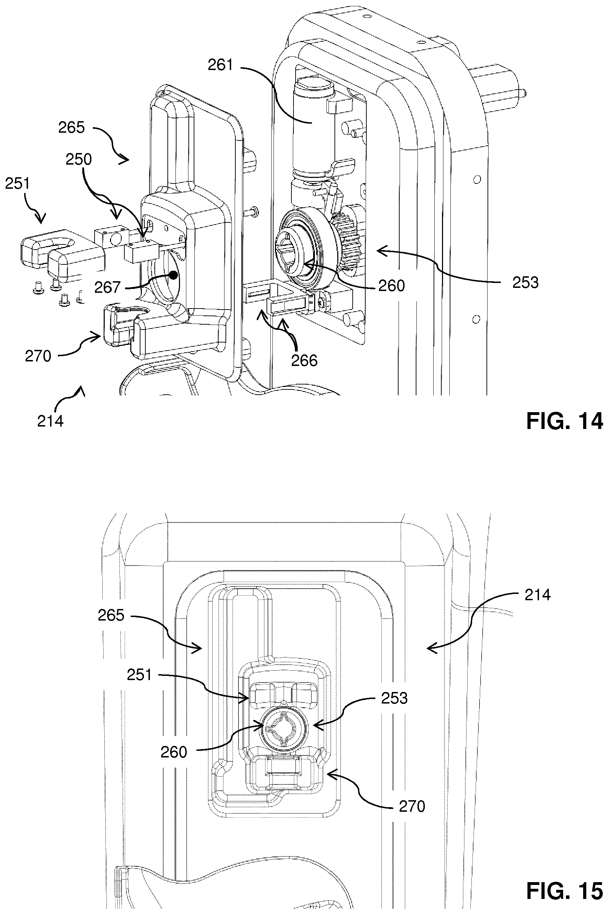

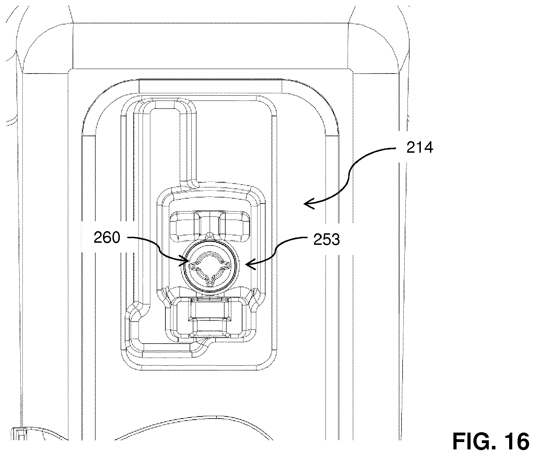

The syringe (241) also can include a plunger that can be slidably received into an internal volume of the syringe body. A plunger flange can be positioned on the plunger at one of the end portions. Referring to the FIG. 1-5, a transfer station (214) is configured to transfer fluid from a source fluid container (240) (i.e., a vial), through the connector (253) into which a fluid switch (252) attachable, and into a syringe (241) when the syringe plunger is retracted. A syringe body of the syringe (241) can include a body flange positioned at the end of the body opposite a tip of the syringe. In that position, said connector (253) only allows fluid transfer between the source fluid connector (240) and the syringe (241), the position of the connector (253) is as shown in FIG. 15. When the syringe plunger is advanced, the medical fluid can be driven out of the syringe (241), through the fluid switch (252), an IV bag hose (171) and into an IV bag (170) or the target container wherein the position of the connector (253) is as shown in FIG. 16. The fluid transfer system (100) includes a mounting module (210) configured to receive the syringe (241) having different size and shape. The mounting module (210) has means which can engage to the syringe (241) so that a motor (e.g., servo, DC, and a step motor) can precisely retract and advance the syringe plunger to transfer the fluid from the source fluid connector (240) to the IV bag (170).

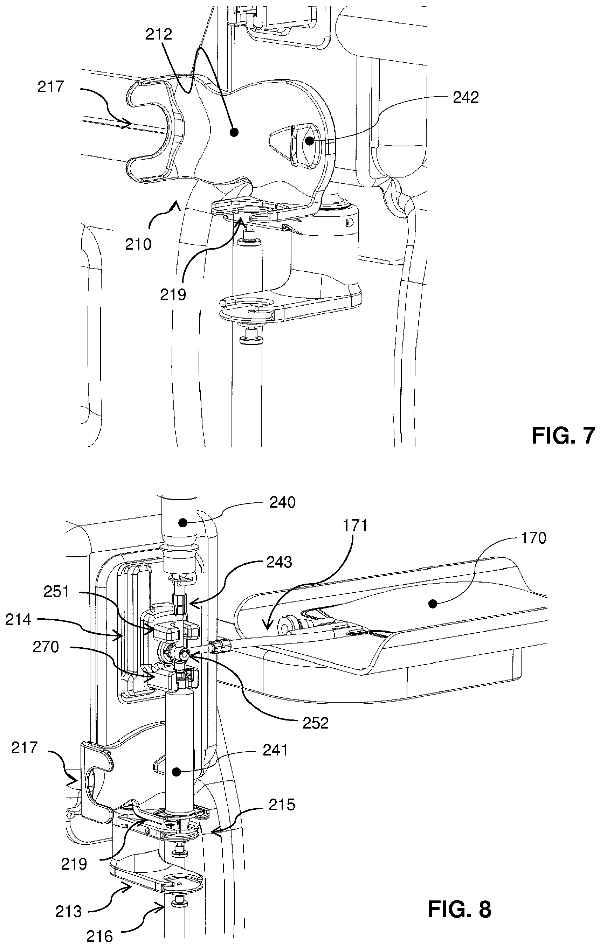

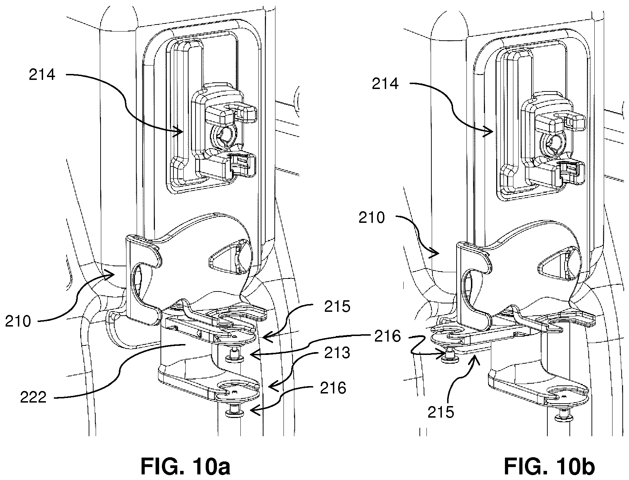

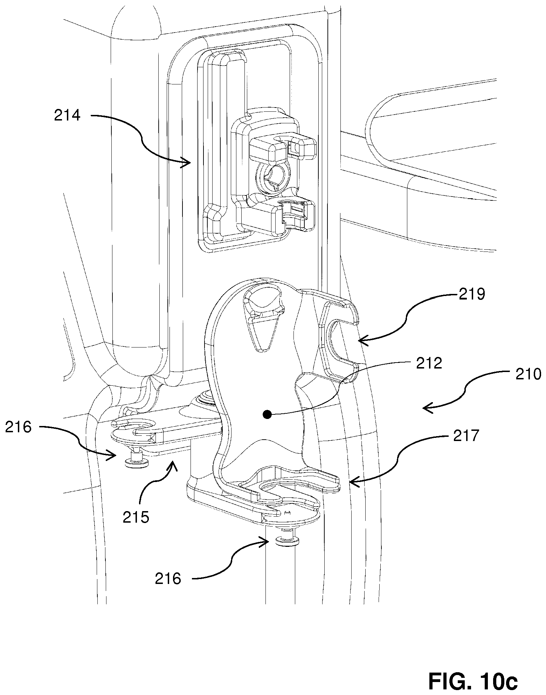

FIG. 1 illustrates the transfer station (214) with the connector (253) and the syringe (241) secured thereto by the mounting module (210). The mounting module (210) includes selector lever (212) which can be rotated around an axis for allowing to hold the syringe (241) with other size and/or shape, as shown in FIG. 10c. Said selector lever (212) can include a first mounting portion (217) and a second mounting portion (219). As shown in FIG. 8, the syringe (241) and the third engagement portion of the fluid switch in an engaged configuration, and said first mounting portion (219) is used to hold the small size syringe (241) (i.e., 20/30 ml. type syringe). In a similar manner, said second mounting portion (217) is arranged to hold the large size syringe (241), such as 50/60 ml. In the illustrated embodiment, a rotatable upper flange housing (215) is placed under the selector lever (212). When the user want to use the larger size syringe (241), said upper flange housing (215) is rotated as shown in FIG. 10b. At the next step, said mounting module (210) is rotated and the first mounting portion (217) of the selector lever (212) is moved to remain on top of a lower flange housing (213).

In a preferred embodiment, said lower flange housing (213) is shaped like the upper flange housing (215). Both the upper flange housing (215) and the lower flange housing (213) are configured to receive a portion of the syringe (241) such as a flange of the syringe body. The upper flange housing (215) and the lower flange housing (213) can be used as an actuator for engaging the plunger of the syringe (241) (e.g., by a plunger flange). Said upper flange housing (215) and the lower flange housing (213) can be driven by the motor (290) (e.g., step motor, DC motor, Servo motor) so that the housings (215, 213) moves with respect to the first mounting portion (217) or the second mounting portion (219). By moving the upper flange housing (215) and the lower flange housing (213) downwardly, away from the first mounting portion (217) or the second mounting portion (219)., the plunger can be withdrawn to draw fluid into the syringe (241). By moving the upper flange housing (215) and the lower flange housing (213) upwardly, towards the first mounting portion (217) or the second mounting portion (219), the plunger can drive the fluid out of the syringe (241). In the prior art, such automated systems are capable of only using one type syringe (241) to transfer the medical fluid. It is possible to use syringes of different sizes and shapes with the present invention.

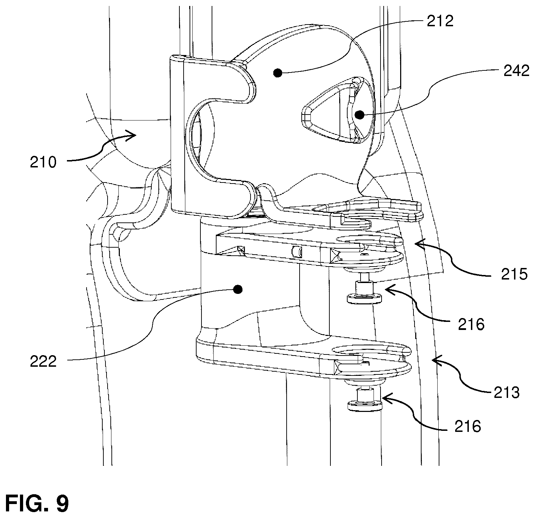

In some embodiments, both the upper flange housing (215) and the lower flange housing (213) can include at least one fixing member (216) for fixing the flange portion of the plunger of the syringe properly. An upper fixing member (216) can be attached to underneath of the upper flange housing (215) as shown in FIG. 9., likewise, a fixing member (216) can be attached to underneath of the lower flange housing (213) as shown in FIG. 9. Said upper and lower fixing members (216) can be used for applying pressure to the plunger flange and prevent the syringe (241) from accidentally disengaging from the mounting module (210). An intermediate portion (222) extends between the upper flange housing (215) and the lower flange housing (213).

In a preferred embodiment, mainly four different types of serum bags, 100 ml, 250 ml, 500 ml and 1000 ml can be used for medical fluid transfer. After selecting the size of the serum bag through the user interface (140), the selected selection is transferred to the database and the information is displayed on the screen as a warning. In this warning, the user is asked to take the bulk serum and make the hose assembly (311) connection. The user is allowed to change the amount, but the volume of serum to be transferred cannot exceed the volume. The changed amount information is transferred to the data bank of controller of the system (100).

The fluid transfer system (100) should be warned by the system that the hose assembly (311) in use should be replaced when the serum type needs to be changed, and should allow transfusion after approval by the user. When the transfer process is completed, a warning appears on the screen. With this warning, the fluid transfer system (100) generates a label with square label printed on the information, which also provides the patient name and bag content (amount and serum type) in writing and also the information to be accessed from the information bank.

A transfer station (214) can be configured to transfer fluid from the source fluid container (240) to the serum bag (170). The fluid is transferred from the source fluid container (240) through a fluid switch (252), and into the IV bag (170). Said transfer station (214) includes the connector (253) for attaching a fluid switch (252) therein and a plurality of detector and sensor with their housings and a cover (265). Said presence detector housing (270) is formed on the cover (265), said cover (265) is configured to cover the connector (253) and the motor (261).

The control system including system software in the fluid transfer system (100) groups the patients to be treated, the medicaments and patient appointments during the day. Patients use the same medicament is grouped in the same group. The user see the medicament and appointments on the screen where they are guided by selecting medicament groups at the screen. The medicament group to be treated is selected at the screen and the medicament group is assigned to the fluid transfer system (100) that made the selection and this assignment is forwarded to the data bank. When this group list is displayed on another device, the user will see which device number the previously selected group is assigned to. If desired, the same group is selected and processing continues. When the same group is selected, the operation is not performed for the patient who has started the operation on the other fluid transfer device (100). The information on the data bank provides the information to the user on the screen that the other patient is in operation. Apart from the operation with the group on the screen, it can be provided with a separate screen which allows selection of the patients through the list of the treatment recipients within that day. Information about the patient selected from within the group or out of the group is displayed on the screen of the user interface (140).

Retrieved data can contain protocol number, patient name, name of the medical fluid/medicament, the amount of the medicament to be used and the amount and type of IV serum to be transferred. If the IV serum bag (170) is prepared with pre-fill or pre-bag for the selected patient previously, it will be indicated on the screen that the when the patient is selected. As mentioned before, the label on the IV bag (170) can include information such as the fluid type, the concentration the amount of fluid, the intended patient, the requesting doctor, etc. If there is no pre-fill, the pre-fill procedure for the patient is selected on the screen and the pre-fill procedure is achieved with the positive displacement pump (150).

The system (100) also allows the use of ready-to-use serum bags provided with full serum. If pre-filling is not done, it is confirmed on the screen that the ready-made serum bag, which is filled with serum, is to be used for this patient. On the screen, the types of serum that are relevant to the medical fluid to be used are listed. The user selects the serum bag content from the listed menu. How much serum is needed for the medicament is also given to the user on the screen. The printer (160) can print a label automatically to be placed on an IV bag (170) and said label - suitable to be read with the barcode reader- is attached onto the relevant place. Since the patient is selected, the serum bag and the patient information are recorded as being matched in the data bank.

The system (100) indicates what type of syringe (241) is used according to the amount of medicament to be transferred. According to the patient's data, the fluid transfer system (100) recommends a larger syringe (241) such as 60 ml instead of a 20 ml syringe if 40 ml of fluid (e.g., chemotherapy drug or other medication) is to be transferred. If 10 ml of fluid (e.g., chemotherapy drug or other medication) is to be transferred, 20 ml syringe (241) is recommended. The user can select either the syringe (241) on the screen according to the stock status or the available syringe (241) in hand.

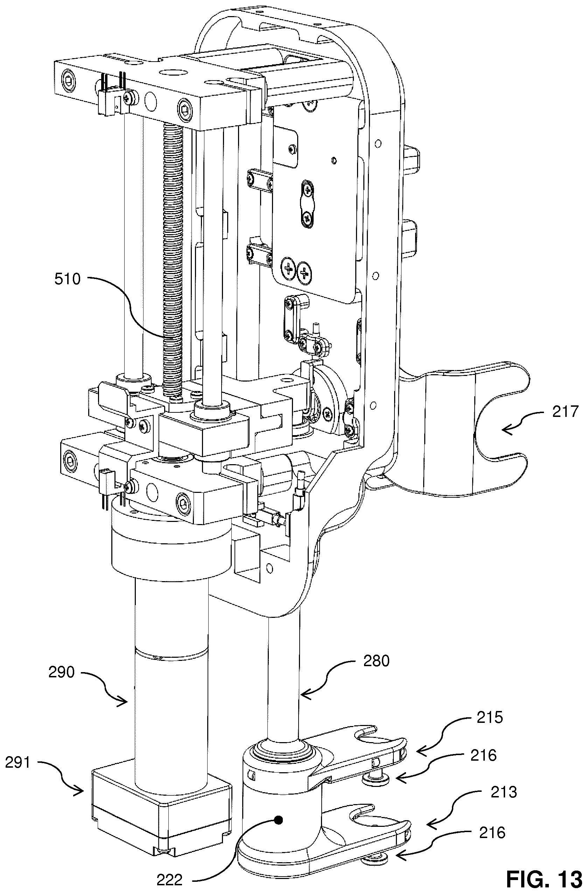

When the system is turned on, as shown in FIG. 13 and 14, a connector motor (261) for actuating the connector (253) and another motor (290) for actuating a plunger actuator (280) are in the home position. Said connector motor (290) is used with motor means (i.e., a wheel) to actuating the connector (253). For example, if the user chooses a syringe (241) having a 20 ml for use, the position of the selector lever (212) is brought into a position for holding the smaller size syringe (241) as shown in FIG. 7. Said selector lever (212) can be controlled by a selector lever lock (242) located at an end portion of the selector lever (212) by sensing the position of the syringe (241). If the selector lever lock (242) does not sense any syringe (241) therein (such as 20 ml syringe barrel), the fluid transfer system (100) warns the user through the user interface (140). When the syringe (241) is brought into the proper position, as shown in FIG. 1, the next step is taken. The selector lever (212) can include two magnet sensor for sensing what type of the syringe (241) is attached. When smaller one such 20 ml syringe (212) is selected, it is checked whether one of the magnet sensors active or not. If it is not active, the system warns the user through the user interface (140).

When the user wants to select larger syringe (241) such as 60 ml, the selector lever (212) is brought into a position as shown in FIG. 10c after following the steps as shown FIG. 10a and 10b, respectively. The position of the selector lever (212) is controlled by the one of the magnet sensors, if the sensor is not active, i.e. not in the 60 ml position, the fluid transfer system (100) warns the user via the user interface (140). At the next step, the system (100) prompts the user to read the qr code of the medicament to the barcode reader. When the system read the said qr code by the barcode reader, information is retrieved including the patient's name, surname, the name of the medicament, and the amount of the medicament to be transferred. As a result of this reading, the medicament is matched with the patient data and transferred to the data bank.

The user interface (140) alerts the user to replace the syringe (241), the fluid switch (252) and source fluid container (240) attached to the source connector portion (243). The user inserts the syringe (241) in place and presses the confirmation button. When the syringe (241) is placed to its housing, a presence detector (266) provided at the mounting module sense the syringe (241), if said presence detector (266) does not sense the syringe (241), the system warns the user. After approval; the user is prompted to put the IV bag (170) on the tray of measuring unit (130) and to make the connection with fluid switch (252) through IV bag hose (171). After the user approval, weighing process is performed and related information is transferred to the information ban. If the user approves the IV bag (170) without putting it on the measuring unit (130), it should be determined that there is no weight on the tray and return to the same warning screen.

The syringe (241) can be a simple reciprocating pump consisting of a plunger (actually a piston) that fits tightly within a cylindrical tube (called a barrel). According to the present invention, as shown in FIG. 13, the plunger of the syringe (241) can be adjusted by a shaft (510) with a piston stroke (screw pitch) of 1, 2 or 3 mm. Said shaft (510) is actuated with the motor (290) which is in a communication with a controller (291). Said motor (290) can be used with a gear box having a reduction of 1/7. As an example, for 20 ml. syringes, to obtain or push around 1 ml, 3,5 mm axial movement is need. For a 60 ml syringe (241), it is required a 1.8 mm an axial movement. Since the syringe (241) attached to the system (100) is known, the system (100) calculates the axial movement value corresponding to the amount of medicament to be transferred, using the axial movement value required for 1 ml. transfer of the syringe (241). The number of turns of the threaded shaft (510) is calculated by using the screw pitch value according to the obtained axial movement value. Said threaded shaft (241) is connected to the reduction output of the motor (290).

Referring to the FIG. 14, when the fluid transfer is started, at least one fluid detector (250) continuously check the presence of the fluid. In a possible embodiment, two fluid detectors (250) placed in mutually opposing relation are closed with a fluid detector housing (251) having U-shaped cross section. A fluid switch (252) can provide fluid communication to the source fluid container (240). The fluid switch (252) can be a stopcock or any other switch that can be actuated between at least two configurations. In a preferred embodiment, a stopcock is used as the fluid switch (252), said stopcock includes three-way flow directions configuration.

After the operation has started, the fluid detector (250) checks the fluid presence after 1 ml of exposure. If the fluid detector (250) sense the presence of the fluid, the process continues. If there is still no medical fluid signal after 1 ml is injected, the process is stopped. The transfer ends when the desired amount of medical fluid is drawn or when the fluid detector (250) indicates that no liquid is present. Source fluid container (240) and fluid switch (252) connections are checked when the user removes the syringe (241) from the system (100). After confirming that the syringe (241) has been removed, the system (100) returns to the home position. Before starting to transfer of the medical fluid, the volume of the attached source fluid container (240) needs to be known. It is calculated how much of the source fluid container (240) can be transfer considering the amount of fluid to be transferred. For this, the volume information of the medical fluid related to the attached the source fluid container (240) in the data bank is reached. If the source fluid container (240) is used before, the remaining amount of the source fluid container (240) can also be used. Amount of fluid remaining in the source fluid container (240) is used to prevent the syringe (241) from taking too much than it is desired. For example, the amount of medical fluid to be given to the patient is 8 ml. and the content of the source fluid container (240) is 5 ml. The software knows it is not possible to transfer more than 5ml. The flow of liquid is controlled by the fluid detector (250) when the transfer is started. For example, the fluid detector (250) can stop the transfer before drawing about 5 ml from the source fluid container (240) and reports to the user on the screen. In both cases, the transferred amount is deducted from the source fluid container (240) and the data is recorded on the data bank of the system (100). The amount transferred into the syringe (241) is transferred to the IV bag (170) through the fluid switch (252), and the remaining amount can be transferred by the same way.

Referring to the FIG. 16, a female shaft (260) of a connector (253) for the fluid switch (i.e., stopcock) is rotated (i.e., 90°) to allow fluid transfer between the syringe (241) and the IV bag (170). When the transfer from syringe (241) to the IV bag (170) is done, said female shaft (260) of the connector (253) is rotated to its "home position" as shown in FIG. 15. When the specified medical fluid amount to be transferred is enough, a new source fluid container (240) can be attached. After that, the IV bag (170) into which the medical fluid transferred is measured and the change of which is calculated by the measuring unit (130). Final weight information is transmitted to the fluid transfer system (100). If the change is within ± 5% of the amount of medication to be given to the patient, the process is terminated successfully.

In a preferred embodiment of the present invention, the fluid transfer system (100) includes two motors, one of which is the motor (290) for actuating the threaded shaft (510) then actuating the tubular-like plunger actuator (280) through which the plunger of the syringe (241) is pushed or retracted, and the other one is the connector motor (261) for arranging the position of connector (253) into which the fluid switch (252) is placed. When the system is opened, said motor (290) and connector motor (261) are brought into the home position. To bring into system (100) the home position, the syringe (241) and fluid switch (252) should not be placed to the fluid transfer station (214). If it is not, at least one presence detector (266) senses and warns the user through the user interface (140) to displace the syringe (241) and fluid switch (252). In a possible embodiment, two presence detectors (266) placed in mutually opposing relation are closed with a presence detector housing (270) having a substantially U-shaped cross section.

In a possible embodiment, there are two different positions for the connector (253) in which the female shaft (260) is rotated. The home position of the connector (253) is as shown in FIG. 15, when the female shaft (260) is rotated in the clockwise direction the fluid switch (252) allows to transfer of the medical fluid from the syringe (241) to the IV bag (170). When the machine is turned on for the first time, the connector (253) is brought to the home position.

## Claims

1. A medical fluid transfer system (100) for use in a fluid transfer process that transfers selected amounts of fluid from a source fluid container (240) to an IV bag (170) comprising:
an user interface (140) configured to receive instructions from an user;
a transfer station (214) having a connector (253) configured to couple to a fluid switch (252) for controlling fluid flow;
a motor (261) for arranging the position of the connector (253);
a control system configure to draw at least a portion of the fluid from the source fluid container (240) by retracting a plunger on the syringe (241), and advance the plunger of the syringe (241) to drive at least a portion of the fluid towards the IV bag (170) **characterized in that** said medical fluid transfer system (100) further comprises:
a mounting module (210) for holding and actuating the syringe (241), wherein the mounting module (210) includes a selector lever (212) with at least two mounting portions which can be selected with respect to the size of the syringe (241), an upper flange housing (215) configured to receive a portion of a plunger of the syringe (241), and a lower flange housing (213) configured to receive a portion of the plunger of the syringe (241); and
a motor (290) which is in communication with the upper flange housing (215) and the lower flange housing (213) for actuating the plunger of the syringe (241), **in that** said upper flange housing (215) and the selector lever (212) are movable such that the syringe (241) at a different size can be used in the medical fluid transfer system (100).

2. A medical fluid transfer system (100) according to Claim 1, wherein the mounting module (210) comprises at least one fluid detector (250) configured to detect a presence or absence of fluid in the fluid switch (252) in that said at least one fluid detector (250) is provided with a fluid detector housing (251).

3. A medical fluid transfer system (100) according to Claim 1 or 2, wherein the mounting module (210) comprises at least one presence detector (266) configured to detect a presence or absence of the syringe (241) in that said at least one presence detector (266) is provided with a presence detector housing (270).

4. A medical fluid transfer system (100) according any one of the Claims 1 to 3, wherein the connector (253) comprises a female shaft (260) which can be brought into two different positions wherein a first position of the connector (253) is configured to allow fluid to flow from the source fluid container (240) to the syringe (241) and block fluid from flowing from the syringe (241) to the source fluid container (240), a second position of the connector (253) is configured to allow fluid to flow from the syringe (241) to the IV bag (170) and block fluid from flowing from the IV bag (170) to the syringe (241).

5. A medical fluid transfer system (100) according any one of the Claims 1 to 4, wherein the selector lever (212) comprises a first mounting portion (217) and a second mounting portion (219) wherein the first mounting portion (217) and the second mounting portion (219) have an U shaped portion such that, in use, a body flange of the syringe (241) can sit on said U shaped portion.

6. A medical fluid transfer system (100) according any one of the Claims 1 to 5, wherein each of the upper flange housing (215) and the lower flange housing (213) has a fixing member (216) configure to apply pressure to a plunger flange of the syringe (241) and prevent the syringe (241) from disengaging from the mounting module (210).

7. A medical fluid transfer system (100) according any one of the Claims 1 to 6, wherein the medical fluid transfer system (100) further comprises a shaker (120) configured to mix, blend, or to agitate substances in the source fluid container (240) by shaking.

8. A medical fluid transfer system (100) according Claim 7, wherein the shaker (120) has a mounting bracket that allows the source fluid container (240) to be held.

9. A medical fluid transfer system (100) according any one of the Claims 1 to 8, wherein the medical fluid transfer system (100) further comprises a positive displacement pump (150) for transferring of fluid between the first fluid container (300) and the second fluid container (310) or the source fluid container (240), having a casing, a mounting interface (151), a plurality of lobes, a rotor, and a pump motor wherein said positive displacement pump (150) is configured to use with a hose assembly (311) such that when the rotor of the positive displacement pump (150) turns, a part of a tube of the hose assembly (311) under compression is compressed, or occludes, thus forcing the fluid to be pumped to move through the tube.

10. A medical fluid transfer system (100) according to Claim 9, wherein the positive displacement pump (150) is a peristaltic pump.

11. A medical fluid transfer system (100) according any one of the Claims 1 to 10, wherein the selector lever lock (242) comprises a selector lever lock (242) configured to sense a presence or absence of the syringe (241) in the selector lever lock (242) wherein if the selector lever lock (242) does not sense the presence of the syringe (241) the medical fluid transfer system (100) warns the user through the user interface (140).

12. A medical fluid transfer system (100) according any one of the Claims 1 to 11, wherein said fluid switch (252) has a first engagement interface configured to couple to the source fluid container (240), a second engagement interface coupled to a IV bag hose (171) and a third engagement interface coupled to a syringe (241).

13. A medical fluid transfer system (100) according any one of the Claims 1 to 12, wherein the selector lever (212) comprises at least two magnet sensor for sensing what type of the syringe (241) is attached to the selector lever (212).

14. A method of transferring fluid using a medical fluid transfer system (100), the method comprising:
receiving instructions through an user interface (140) of the medical fluid transfer system (100), the instructions identifying a specified volume of fluid to transfer from a source fluid container (240) to a IV bag (170);
moving an upper flange housing (215) and/or a selector lever (212) of a mounting module (210) with respect to the size of the syringe (241);
providing a fluid switch (252) to couple to a connector (253) of the medical fluid transfer system (100) for controlling fluid flow, said connector (253) comprising a female shaft (260) movable between a first position and a second position;
attaching the source fluid container (240), the syringe (241) and the IV bag (170) to the fluid switch (252);
transferring the fluid from the source fluid container (240) to the IV bag (170) by a control system of the medical fluid transfer system (100),
stopping the transfer of the fluid when the specified volume of fluid is transferred to the IV bag (170) based on the information received from the user interface (140) or stopping the transfer of the fluid based on the information received from a fluid detector (250) configured to detect a presence or absence of the fluid in the fluid switch (252).

15. A method according to the Claim 14, further comprising attaching a plunger of the syringe (241) to a lower flange housing (213) or an upper flange housing (215) on the fluid transfer system (100) with respect to the type of selected syringe (241), the lower flange housing (213) and the upper flange housing (215) being coupled to a motor (290) and configured to move the plunger of the syringe (241) to transfer fluid from a the source fluid container (240) through the fluid switch (252) and into the syringe (241) as the plunger is withdrawn and to transfer fluid from the syringe (241) through the fluid switch (252) and into the IV bag (170) as the plunger is advanced.
